# EUROPÄISCHE PATENTANMELDUNG

(11) **EP 1 884 192 A1**
(43) Veröffentlichungstag der Anmeldung: **06.02.2008**
(21) Anmeldenummer: 06016389.6
(22) Anmeldetag: 05.08.2006
(51) Int. Cl.: A61B 5/151

(54) **Verfahren zum Herstellen von Lanzetten zur Entnahme von Blut und Lanzette mit einer geschliffenen Spitze**

(71) Anmelder: Roche Diagnostics GmbH, 68305 Mannheim (DE); F.HOFFMANN-LA ROCHE AG, 4070 Basel (CH)
(72) Erfinder: Weiss, Thomas, 68307 Mannheim (DE)
(74) Vertreter: Pfeifer, Hans-Peter

(57) **Zusammenfassung**

Verfahren zum Herstellen von Lanzetten zur Entnahme von Blut für medizinisch-analytische Zwecke, die wenigstens eine Schlifffläche (31) aufweist, die in einem Schleifvorgang erzeugt wird, wobei ein Lanzettendraht (6) mittels einer Positioniereinrichtung (2a) in einer definierten Schleifposition relativ zu einem Schleifelement (10) einer Schleifeinrichtung (1) positioniert wird, dadurch gekennzeichnet, dass die folgenden Verfahrensschritte in dieser Reihenfolge durchgeführt werden: Abziehen des Lanzettendrahtes (6) von einer Rolle (5) und transportieren in die Positioniereinrichtung (2a); Fixieren des Lanzettendrahtes (6) in der Positioniereinrichtung (2a); Schleifen des freien Endes (7) des Lanzettendrahtes (6) mittels der Schleifeinrichtung (1) zum Erzeugen wenigstens einer Schlifffläche (31), und Abtrennen des freien Endes (7) des Lanzettendrahtes (6) in einer Trennposition zu einer Lanzette (28) mit definierter Länge.

## Beschreibung

Die Erfindung betrifft ein Verfahren zum Herstellen von Lanzetten zur Entnahme von Blut für medizinisch-analytische Zwecke. Die Lanzetten haben eine Schlifffläche, die in einem Schleifvorgang erzeugt wird, wobei ein Lanzettendraht mittels einer Positioniereinrichtung in einer definierten Schleifposition relativ zu einem Schleifelement einer Schleifeinrichtung positioniert wird. Die Erfindung betrifft auch eine Lanzette aus Lanzettendraht.

Lanzetten zur Entnahme von Blut aus einem Körperteil werden in der medizinischen Analytik verwendet, um eine Wunde in einem Körperteil zu erzeugen, aus der Blut austritt. Die Lanzetten werden bevorzugt in Stechgeräten oder Stechhilfen eingesetzt, mit denen sich der Patient selbst stechen kann.

Derartige Stechhilfen werden im Bereich der Diabetis Care eingesetzt. Hier ist es von besonderer medizinischer Bedeutung, dass die Patienten den Blutzuckergehalt ihres Blutes regelmäßig kontrollieren. Nur so ist eine lückenlose und durchgängige Überwachung der Insulinwerte des Patienten möglich, um stets eine angepasste, optimale Einstellung der Insulingabe zu ermöglichen. Dadurch werden Spätfolgen starker Blutzuckerschwankungen, wie beispielsweise Blindheit, vermieden. Studien haben gezeigt, dass die Akzeptanz und Anwendung der Stechhilfen von dem Einstichschmerz abhängt. Deshalb wird eine häufige Kontrolle von dem Patienten nur dann durchgeführt, wenn das Schmerzempfinden beim Einstich in die Haut möglichst klein ist.

Ein Faktor, der das Schmerzempfinden der Patienten wesentlich bestimmt, ist die Einstichtiefe. Um eine exakt reproduzierbare Stechtiefe zu gewährleisten, sollen die Lanzetten exakt eine vorbestimmte Länge haben. Auch beim Auswechseln einer Lanzette und beim Ersatz einer gebrauchten Lanzette durch eine neue müssen alle neuen Lanzetten die gleiche Länge haben. Deshalb ist die zulässige Längentoleranz der Lanzetten sehr gering, was die Herstellung von Lanzetten mit bisherigen Verfahren verteuert.

Das Schmerzempfinden der Patienten wird auch wesentlich von der Qualität der geschliffenen Spitze beeinflusst. Als besonders geeignet hat sich ein Dreifacettenschliff der Lanzettenspitze erwiesen, bei dem eine Grundfläche und zwei zueinander geneigte Schneidflächen vorhanden sind, so dass eine exakte und feine Spitze am Lanzettenende entsteht. Zwischen den Schneidflächen und der Mantelfläche der Lanzette wird jeweils eine scharfe Schneide gebildet, die die Haut aufschneidet. Beim Einstich wird in einer ersten Phase von der Spitze ein kleines Loch in der Haut erzeugt. Beim weiteren Eindringen der Lanzette in die Haut schneidet die Schneidefläche die zunächst kleine Öffnung weiter auf, so dass nach Herausziehen der Lanzette aus der Haut Blut austreten kann.

Zur Herstellung derartiger Lanzetten wird Lanzettendraht auf die gewünschte Länge zugeschnitten. Danach wird ihre Spitze geschliffen. Dazu werden die Lanzettendrahtstücke auf einem Klemmbrett montiert, auf dem sie während des Schleifvorgangs gehalten werden. Zur Erzeugung der einzelnen Schliffflächen werden zwei Klemmschienen des Klemmbrettes gegeneinander verschoben, so dass die einzelnen Lanzetten um ihre jeweilige Längsachse gedreht werden. Der Drehwinkel, der mit einer derartigen Anordnung erreicht werden kann, liegt in einem Bereich von kleiner 90°. Nur bei solchen kleinen Drehwinkeln ist es möglich, mehrere nebeneinander angeordnete Lanzetten gleichmäßig um die gleiche Winkelposition zu verdrehen. Sowohl das Abschneiden der Lanzettendrahtstücke als auch das Einspannen in dem Klemmbrett müssen mit hoher Genauigkeit vorgenommen werden. Nur so lassen sich Lanzetten mit exakter Länge und geringer Fertigungstoleranz herstellen. Das sehr präzise Vorgehen in den beiden Herstellungsschritten macht die Gesamtherstellung der Lanzetten zeitaufwendig und teuer. Eine Massenproduktion ist nur schwer möglich. Der Anteil an manuellen Eingriffen und Überwachungen im Herstellungsprozess ist relativ hoch.

Da sich die Diabetespatienten mehrmals am Tag stechen müssen, um eine kontinuierliche Kontrolle ihrer Blutzuckerwerte durchführen zu können, werden immer häufiger Stechgeräte verwendet, die ein Lanzettenmagazin mit mehreren Lanzetten enthalten. Damit der Patient das Stechgerät auf komfortable Weise mit sich führen kann, muss es möglichst klein sein. Dies führt zu der Forderung, dass auch die verwendeten Lanzetten möglichst klein und kurz sein sollen. Die Länge der Lanzetten, die mit den bisher bekannten, oben beschriebenen Verfahren hergestellt werden können, ist jedoch auf ein Mindestmaß begrenzt. In der Praxis ist eine Herstellung von Lanzetten mit einer Länge kleiner als 16 mm nicht möglich, vor allem weil die Lanzetten in dem als Dreh- und Positioniereinrichtung dienenden Klemmbrett während des Schleifvorgangs nicht mehr exakt gehalten werden.

Aus hygienischen Gründen sollen die Lanzetten nur einmal verwendet werden. Deshalb besteht das Bestreben, sie möglichst kostengünstig herzustellen. Dies führt zu dem Wunsch einer Automatisierung des Herstellungsprozesses, um in Großserien produzieren zu können.

Aufgabe der vorliegenden Erfindung ist es deshalb, ein verbessertes Verfahren zur Herstellung von Lanzetten mit einer geschliffenen Spitze vorzuschlagen, welches sich insbesondere auch im Großserieneinsatz verwirklichen lässt.

Gelöst wird die Aufgabe durch ein Verfahren mit den Merkmalen des Anspruchs 1. Bei dem erfindungsgemäßen Verfahren zum Herstellen von Lanzetten zur Entnahme von Blut für medizinisch-analytische Zwecke, die eine erste Schlifffläche aufweisen, die in wenigstens einem Schleifvorgang erzeugt wird, wird ein Lanzettendraht mittels einer Positioniereinrichtung in einer definierten Schleifposition relativ zu einem Schleifelement einer Schleifeinrichtung positioniert. Zum Herstellen der Lanzetten wird der Lanzettendraht von einer Rolle abgezogen und in die Positioniereinrichtung transportiert, in der er anschließend fixiert wird. In einem weiteren Schritt wird das freie Ende des Lanzettendrahtes mittels einer Schleifeinrichtung geschliffen, um die Schlifffläche zu erzeugen. Erst nach dem Schleifen der Schlifffläche wird das freie Ende des Lanzettendrahtes an einer Trennposition abgetrennt, wodurch eine Lanzette mit einer definierten Länge entsteht.

Nach diesem Verfahren wird also, im Gegensatz zu den bekannten Verfahren, erst ein freies Ende des Lanzettendrahtes geschliffen und anschließend der Lanzettendraht in der gewünschten Länge abgeschnitten. Auf diese Weise lassen sich Lanzetten mit einer sehr exakten Länge herstellen. Die Längentoleranzen sind sehr gering. Wird beispielsweise der Lanzettendraht zu weit nach vorne geschoben, so dass der Abstand zwischen Lanzettendraht und Trennposition größer ist als die gewünschte Länge der Lanzette, so wird durch die Schleifeinrichtung das freie Ende des Lanzettendrahtes so weit abgeschliffen, dass die zwischen der Schleifeinrichtung und der Trennposition eingestellte Länge resultiert. Die Länge der hergestellten Lanzette hängt also nicht vom Vorschub des Lanzettendrahtes, sondern lediglich von der Trennposition und deren Abstand von der Schleifeinrichtung ab. Durch einfaches Justieren lässt sich die gewünschte Länge der Lanzetten einstellen.

Da der Lanzettendraht von einer Rolle abgewickelt wird, liegt er quasi als Endlosdraht vor. Sobald das freie Ende des Lanzettendrahtes geschliffen und nach dem Schleifen die Lanzette abgetrennt wird, wird der Lanzettendraht in Richtung auf die Positioniereinrichtung weitertransportiert. Somit kann nach einem einzigen einfachen Vorbereitungsschritt sofort mit dem Schleifen des neuen freien Endes des Lanzettendrahtes begonnen werden. Ein derartiger Vorgang lässt sich einfach automatisieren. Das Zuführen des Lanzettendrahtes kann mit hoher Geschwindigkeit erfolgen. Eine exakte Positionierung ist nicht notwendig, weil sie keinen Einfluss auf die spätere Länge der Lanzette hat.

Die Verfahrensschritte Abziehen des Lanzettendrahtes, Transportieren und Fixieren, Schleifen und anschließendes Abtrennen werden in der angegebenen Reihenfolge durchgeführt. Dies schließt selbstverständlich nicht aus, dass vor, nach oder zwischen den genannten Verfahrensschritten weitere zusätzliche Verfahrensschritte durchgeführt werden können.

Bevorzugt wird mit dem Verfahren eine Lanzette mit einer geschliffenen Spitze hergestellt, die eine erste Schlifffläche und mindestens eine weitere Schlifffläche, die in einem definierten Winkel zueinander orientiert sind, aufweisen. Das freie Ende des Lanzettendrahtes wird mittels der Schleifeinrichtung zum Erzeugen der Mehrzahl von Schliffflächen geschliffen. Dabei wird in einer Mehrzahl von Schleifvorgängen jeweils eine der Schliffflächen erzeugt und der Lanzettendraht zwischen den Schleifvorgängen in eine der nächsten zu erzeugenden Schlifffläche entsprechende Schleifposition gedreht, wobei die Drehbewegung eine axiale Drehbewegungskomponente in Bezug auf die Längsachse des Lanzettendrahtes hat. Im einfachsten, Fall hat die Drehbewegung nur eine axiale Komponente, d.h. der Lanzettendraht wird nur um seine Längsachse gedreht. Selbstverständlich sind auch alle anderen Bewegungen davon umfasst, solange auch eine Drehung um die Längsachse stattfindet. Auf diese Weise lassen sich die Lanzetten mit einer geschliffenen Spitze und einer Mehrzahl von Schliffflächen herstellen. Bevorzugt ist dabei besonders eine Lanzette mit einem Dreifacettenschliff, die eine Grundschlifffläche und zwei als Schneidflächen bezeichnete weitere Schliffflächen aufweist.

Um den Lanzettendraht in die entsprechenden Schleifpositionen zu drehen, wird die Positioniereinrichtung gedreht. Alternativ kann auch die Schleifeinrichtung relativ zur Positioniereinrichtung gedreht werden. Besonders bevorzugt ist jedoch eine Positioniereinrichtung, die wenigstens ein Drehelement aufweist, um den Lanzettendraht zu drehen.

In einer bevorzugten Ausführungsform der Erfindung ist die Positioniereinrichtung zur Aufnahme einer Mehrzahl von Lanzettendrähten ausgebildet. Die Lanzettendrähte sind nebeneinander in einer Ebene angeordnet, die quer zur Längsrichtung ihrer freien Enden verläuft. Auch während der Schleifvorgänge sind die Lanzettendrähte vorzugsweise in dieser Ebene fixiert.

Die Lanzettendrähte sind derart nebeneinander angeordnet, dass mehrere Lanzettendrähte gleichzeitig geschliffen werden können. Es lassen sich also in einem Arbeitsschritt parallel mehrere Schliffflächen erzeugen. Dadurch wird die Stückzahl bei der Herstellung der Lanzetten erhöht. Selbstverständlich können die Lanzettendrähte auch nacheinander geschliffen werden.

In einer weiter bevorzugten Ausgestaltung des erfindungsgemäßen Verfahrens wird das Schleifelement während des Schleifvorganges derartig bewegt, dass die freien Ende von mehreren in der Positioniereinrichtung fixierten Lanzettendrähte während der Bewegung nacheinander geschliffen werden. Das Schleifelement wird bevorzugt entlang der nebeneinander angeordneten Lanzettendrähte bewegt, so dass die Lanzettendrähte nacheinander geschliffen werden, wobei auch mehrere Drähte in dem Sinne gleichzeitig geschliffen werden können, dass mit dem Schleifen eines Lanzettendrahtes schon begonnen werden kann, bevor der Schleifvorgang an einem oder mehreren vorhergehenden Lanzettendrähten beendet ist.

In der Großserienfertigung kann die Positioniereinrichtung eine Breite von 2 bis 3 Metern aufweisen, so dass mehrere Hundert Lanzettendrähte nebeneinander angeordnet sind. Auf diese Weise lässt sich eine sehr kostengünstige Massenproduktion von Lanzetten mit einer geschliffenen Spitze verwirklichen.

Nachdem alle in der Positioniereinrichtung gehaltenen bzw. eingespannten Lanzettendrähte in einem Schleifvorgang bearbeitet sind, wobei eine erste Schlifffläche hergestellt worden ist, werden die in der Positioniereinrichtung fixierten Lanzettendrähte, vorzugsweise gleichzeitig, in eine weitere Schleifposition bewegt, die einer weiteren Schlifffläche entspricht. Danach wird das Schleifelement wieder derart an den Lanzettendrähten vorbeibewegt, dass während der Bewegung ein weiterer Schleifvorgang an den freien Enden der Lanzettendrähte durchgeführt wird.

Ein Vorteil des erfindungsgemäßen Verfahrens besteht darin, dass während des Schleifens nicht bereits die relativ kurze Lanzette fixiert und gehalten werden muss, sondern ein längeres Stück des Lanzettendrahtes an dessen freiem Ende, wobei das freie Ende nicht abgeschnittenen sein muss, also noch mit der Rolle verbunden ist. Dadurch ist eine sehr feste, robuste und zuverlässige Fixierung des Drahtes möglich. Auf diese Weise wird auch die exakte Positionierung des freien Endes des Lanzettendrahtes (in Richtung quer zu seiner Längsachse) verbessert, was zu einer sehr hohen Fertigungsqualität und Produktgüte führt und geringe Fertigungstoleranzen ermöglicht.

In einer bevorzugten Ausgestaltung des erfindungsgemäßen Verfahrens wird das freie Ende des geschliffenen Lanzettendrahtes derartig abgetrennt, dass die Länge der resultierenden Lanzette von deren Spitze bis zu der Trennstelle höchstens 20 mm, vorzugsweise höchstens 16 mm beträgt. Besonders bevorzugt wird das freie Ende des Lanzettendrahtes so abgetrennt, dass die resultierende Lanzette eine Länge von höchstens 5 mm aufweist. Mit dem Verfahren lassen sich deshalb deutlich kürzere Lanzetten herstellen, als es mit bisher bekannten automatisierten Verfahren möglich ist. Das erfindungsgemäße Verfahren bildet deshalb die Grundlage für eine automatisierte Massenproduktion in Großserien von kurzen Lanzetten mit einer geschliffenen Spitze und einer Länge von höchstens 16 mm, vorzugsweise höchstens 12 mm, besonders bevorzugt höchstens 10 mm.

Das Verfahren eignet sich zum Herstellen von Lanzetten mit einem Durchmesser von ca. 0,2 mm bis zu 0,8 mm. Die Lanzette kann dabei aus Vollmaterial bestehen. Es ist jedoch auch möglich, Lanzetten auszubilden, die wenigstens teilweise hohl sind. Mit dem Verfahren hergestellte Lanzetten können einen Kapillarkanal enthalten, der auch als einseitig offene Kapillarnut ausgebildet sein kann.

Eine bevorzugte Ausführungsform des erfindungsgemäßen Verfahrens zur Herstellung von Lanzetten mit einer geschliffenen Spitze wird anhand der beigefügten Zeichnungen näher beschrieben. Die darin dargestellten Besonderheiten können einzeln oder in Kombination verwendet werden, um bevorzugte Ausgestaltungen der Erfindung zu schaffen. Es zeigen:
- Fig. 1: eine Prinzipskizze einer Vorrichtung zum Herstellen von Lanzetten mit geschliffener Spitze;
- Fig. 2: eine Detailansicht der Vorrichtung aus Figur 1 in perspektivischer Darstellung;
- Fig. 3: einen Detailschnitt durch einen Teil einer Dreh- und Positioniereinrichtung der Vorrichtung aus Figur 1;
- Fig. 4, 5,: 6 einen Detailschnitt der Dreh- und Positioniereinrichtung aus Figur 3 während verschiedenen Verfahrensschritten;
- Fig. 7: eine mit dem erfindungsgemäßen Verfahren hergestellte Lanzette,
- Fig. 8: einen Querschnitt entlang einer Linie A-A der Lanzette aus Figur 7;
- Fig. 9: einen Querschnitt entlang einer Linie B-B der Lanzette aus Figur 7;
- Fig. 10: eine alternative Ausführungsform einer mit dem erfindungsgemäßen Verfahren hergestellten Lanzette; und
- Fig. 11: einen Querschnitt durch die Lanzette aus Figur 10.

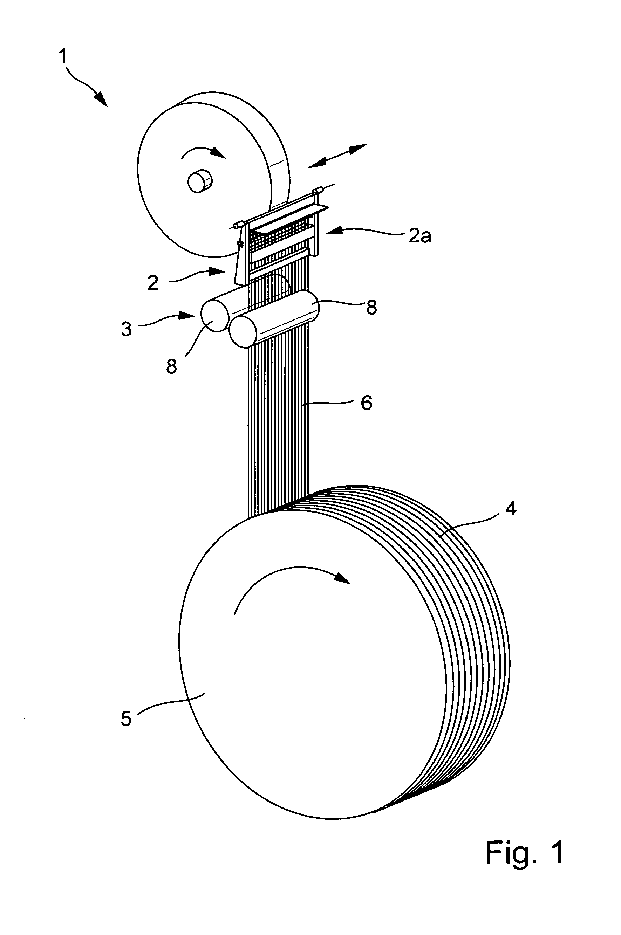

Die Vorrichtung zum Herstellen einer Lanzette mit einer geschliffenen Spitze nach den Figuren 1, 2 und 3 umfasst eine Schleifeinrichtung 1, eine Positioniereinrichtung 2a und eine Transporteinrichtung 3. Die Positioniereinrichtung 2a ist als Dreh- und Positioniereinrichtung 2 ausgebildet, mit der Lanzettendrähte 6 gedreht und positioniert werden können. Die Transporteinrichtung 3 ist zwischen der Dreh- und Positioniereinrichtung 2 und einer als Wickeltrommel 4 ausgebildeten Rolle 5 angeordnet, auf der mehrere nebeneinander angeordnete Lanzettendrähte 6 aufgewickelt sind.

Mittels der Transporteinrichtung 3 werden die parallel angeordneten Lanzettendrähte 6 von der Wickeltrommel 4 abgezogen, in Richtung auf die Dreh- und Positioniereinrichtung 2 transportiert und dieser zugeführt. Jedesmal wenn das freie Ende des Lanzettendrahtes 6 bearbeitet und abgetrennt worden ist, wird der Lanzettendraht 6 weiter in die Positioniereinrichtung 2 nachgeschoben. Die Transporteinrichtung 3 weist dazu zwei sich gegenläufig drehende Transportwalzen 8 auf, deren Drehung einen Transport der Lanzettendrähte 6 bewirkt. Alternativ kann die Transporteinrichtung 3 eine Klemmvorrichtung umfassen, die translatorisch um einen vorbestimmten Hub bewegt wird, um den Lanzettendraht 6 der Positioniereinrichtung 2a zuzuführen. Die Transporteinrichtung 3 kann insbesondere pneumatisch angetrieben sein, um den Vorschub zu realisieren.

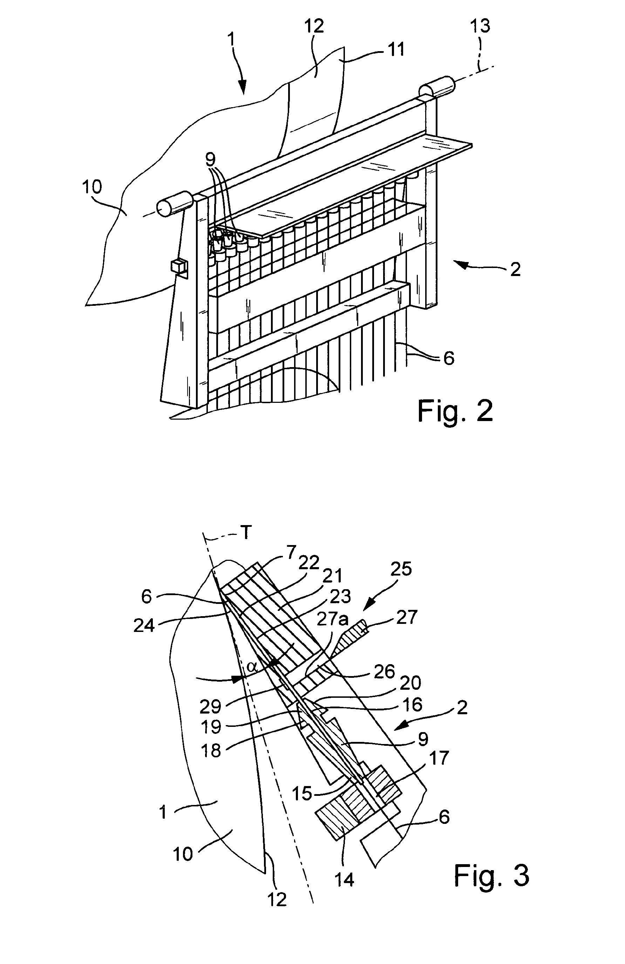

Die in Figur 2 in Detail gezeigte Dreh- und Positioniereinrichtung 2 hat eine rahmenartige Tragkonstruktion. Sie umfasst eine Mehrzahl von Drehelementen 9, die nebeneinander in einer Reihe angeordnet sind. Jedes Drehelement 9 hält jeweils einen Lanzettendraht 6 und fixiert ihn in der Dreh- und Positioniereinrichtung 2.

Die Schleifeinrichtung 1 weist ein Schleifelement 10 auf, das hier als zylindrische Schleifscheibe 11 ausgebildet ist. Das Schleifelement 10 kann auch eine Schleifwalze sein. **Es besteht bevorzugt aus ...(Material).** Die Mantelfläche der Schleifscheibe 11 dient als Schleiffläche 12, die während der Drehung der Schleifscheibe 11 um ihre Mittelachse die freien Enden 7 der Lanzettendrähte 6 schleift. Während der Rotation der Schleifscheibe 11 wird die Schleifeinrichtung 1 quer zu der Längsachse der freien Enden 7 der Lanzettendrähte bewegt, so dass die Dreh- und Positioniereinrichtung 2 entlang ihrer Breite abgefahren wird. Dadurch werden nacheinander alle in der Dreh- und Positioniereinrichtung 2 eingespannten Lanzettendrähte 6 geschliffen (wobei gleichzeitig mehrere Lanzettendrähte 6 bearbeitet werden können). Die Bewegung des Schleifelements 10 findet dabei bevorzugt entlang der Breite der Dreh- und Positioniereinrichtung 2 derart statt, dass das Schleifelement 10 über die Enden der Dreh- und Positioniereinrichtung 2 hinausbewegt wird.

Die Position der Lanzettendrähte 6 in Bezug auf die Schleifeinrichtung 1 bzw. deren Schleiffläche 12 wird durch Neigung der Dreh- und Positioniereinrichtung 2 verändert. Dazu wird die Dreh- und Positioniereinrichtung 2 um eine im Bereich der freien Enden 7 verlaufende Drehachse 13 verschwenkt. Auf diese Weise können an den Lanzettendrähten 6 Schliffflächen mit unterschiedlichen Neigungswinkeln erzeugt werden. Vorzugsweise wird der Neigungswinkel des freien Endes der Lanzettendrähte 6 relativ zu der Schleiffläche 12 des Schleifelements 10 zwischen mindestens zwei der Schleifvorgänge verändert, so dass am Ende des Herstellungsprozesses das freie Ende des Lanzettendrahtes 6 wenigstens zwei Schliffflächen mit unterschiedlichem Neigungswinkel aufweist.

Neben der Neigung der Lanzettendrähte 6 in Bezug auf die Schleiffläche 12 kann auch ihr Drehwinkel, bezogen auf die Längsachse der Drähte, verändert werden. Dazu wird jeder einzelne Lanzettendraht 6 von dem Drehelement 9 der Dreh- und Positioniereinrichtung 2, in dem der Lanzettendraht 6 fixiert wird, durch Drehung des Drehelements 2 um die Längsachse des Lanzettendrahts 6 gedreht.

Das Drehelement 9 ist mittels einer Halterung 14 an der Dreh- und Positioniereinrichtung 2 angeordnet. Es ist vorzugsweise als rotationssymmetrisches, rundes Element ausgebildet, dessen unterer Teil 15 eine konische Form aufweist. Eine Durchgangsbohrung 16 dient zur Aufnahme und Führung des Lanzettendrahtes 6. Zur einfacheren Einführung des Lanzettendrahtes 6 in das Drehelement 9 ist eine untere Öffnung 17 der Durchgangsbohrung 16 aufgeweitet, so dass der Öffnungsdurchmesser im Vergleich zum Durchmesser des Lanzettendrahtes 6 um ein Vielfaches größer ist.

Besonders bevorzugt ist mindestens ein Teil des Drehelements 9 in Längsrichtung des Lanzettendrahts 6 in mindestens drei Segmente 18 segmentiert. Der Lanzettendraht 6 wird durch Zusammendrücken der Segmente 18 in Richtung auf die Achse des Lanzettendrahtes 6 fixiert und gehalten. In der in Figur 3 dargestellten Ausführungsform sind die Segmente 18 im vorderen (in der Figur oberen) Teil 19 des Drehelements 9 angeordnet. Die Segmente 18 sind als Fixierbacken 20 ausgebildet, die den Draht festklemmen, so dass er in seiner fixierten Position durch Drehen des Drehelements 9 um seine eigene Achse, also die Längsachse, gedreht werden kann.

Während des Vorschiebens des Lanzettendrahtes 6 durch die Transporteinrichtung 3 sind die Fixierbacken 20 gelöst, so dass der Draht bewegt werden kann. Dazu kann das Drehelement 9 ähnlich wie die Minenhalterung eines Druckbleistiftes ausgebildet sein, wobei die Fixierbacken 20 durch eine Bewegung in Längsrichtung des Lanzettendrahtes 6 geöffnet bzw. geschlossen werden können.

In Transportrichtung des Drahtes vor dem Drehelement 9 weist die Dreh- und Positioniereinrichtung 2 ein Stützelement 21 auf, das dem freien Ende des Lanzettendrahtes 6 eine Stützfläche 22 bietet, an der der Lanzettendraht 6 während des Schleifvorgangs anliegt. Das Stützelement 21 hat einen Führungskanal 23, der unter einem Schrägwinkel derart durch das Stützelement 21 verläuft, dass der Führungskanal 23 ein offenes Ende 24 aufweist, an dem sich die Stützfläche 22 befindet.

In das Stützelement 21 ist eine Trenneinheit 25 integriert, die eine Nut 26 und ein Schneidmesser 27 umfasst, das in die Nut 26 geführt wird. Die Nut 26 ist derart in dem Stützelement 21 angeordnet, dass der Führungskanal 23 die Nut 26 schneidet. Eine Bodenfläche 29 der Nut 26 fluchtet mit dem Führungskanal 23. Sie bildet während des Abtrennens eine Anlage- und Positionierfläche für den Lanzettendraht 6, der sich durch den Führungskanal 23 erstreckt. Der Abstand der Nut 26 vom oberen Ende des Stützelements 21 ist so gewählt, dass er der gewünschten Lanzettenlänge entspricht. Im gezeigten Beispiel beträgt der Abstand 12 mm. Durch Abtrennen des freien Endes des Lanzettendrahtes 6 mit dem Schneidmesser 27 an einer Trennstelle 27a wird eine Lanzette 28 mit der gewünschten vordefinierten Länge von 12 mm erzeugt. Anstelle der Verwendung des hier gezeigten Schneidmessers 27 kann das freie Ende des Lanzettendrahtes 6 mittels eines Hackmessers, einer Trennscheibe oder eines Lasers abgetrennt werden. Selbstverständlich sind auch weitere bekannte Möglichkeiten zum Durchtrennen eines Drahtes geeignet.

Der Figur 3 ist zu entnehmen, dass der Lanzettendraht 6 vorzugsweise an einer Stelle abgetrennt wird, die zwischen dem Drehelement 9 und der geschliffenen Spitze des Lanzettendrahtes 6 liegt. Mit anderen Worten liegt die Trennstelle 27a zwischen dem Drehelement 9 und dem offenen Ende 24 des Führungskanals 23. Bevorzugt wird das freie Ende 7 des Lanzettendrahtes 6 abgetrennt, während der Lanzettendraht 6 in dem Drehelement 9 fixiert ist. Auf diese Weise wird der Lanzettendraht 6 exakt in seiner Position gehalten, so dass eine glatte Grundfläche entsteht und das nicht geschliffene Ende der Lanzette 28 nicht nachbearbeitet werden muss. Hierdurch werden die Produktionskosten bei der Herstellung der Lanzetten 28 gesenkt.

Zwar wird der Lanzettendraht 6 zwischen dem Drehelement 9 und der geschliffenen Spitze 30 abgetrennt, es kann jedoch auch vorteilhaft sein, den Lanzettendraht von seiner Spitze aus betrachtet hinter den Drehelementen 9 zu durchtrennen. Dies mag unter Fertigungsgesichtspunkten in besonderen Ausführungsformen eine geeignete Variante sein.

Der Neigungswinkel α zwischen der Schleiffläche 12 und dem freien Ende 7 des Lanzettendrahtes 6 kann durch Verschwenken der Dreh- und Positioniereinrichtung 2 verändert werden. Dieser "Schleifwinkel" entspricht dem Winkel zwischen der Achse des freien Endes des Lanzettendrahtes 6 und der im Schleifpunkt gebildeten Tangente T der Schleiffläche 12. Aufgrund der Größe des Schleifelementes 10 im Verhältnis zum Durchmesser des Lanzettendrahtes 6 bzw. zu dessen Spitze kann die Krümmung des Schleifelementes 10 vernachlässigt werden, so dass in erster Näherung die Tangente der Schleiffläche 12 im Schleifpunkt mit der Schleiffläche 12 selbst gleichgesetzt werden kann.

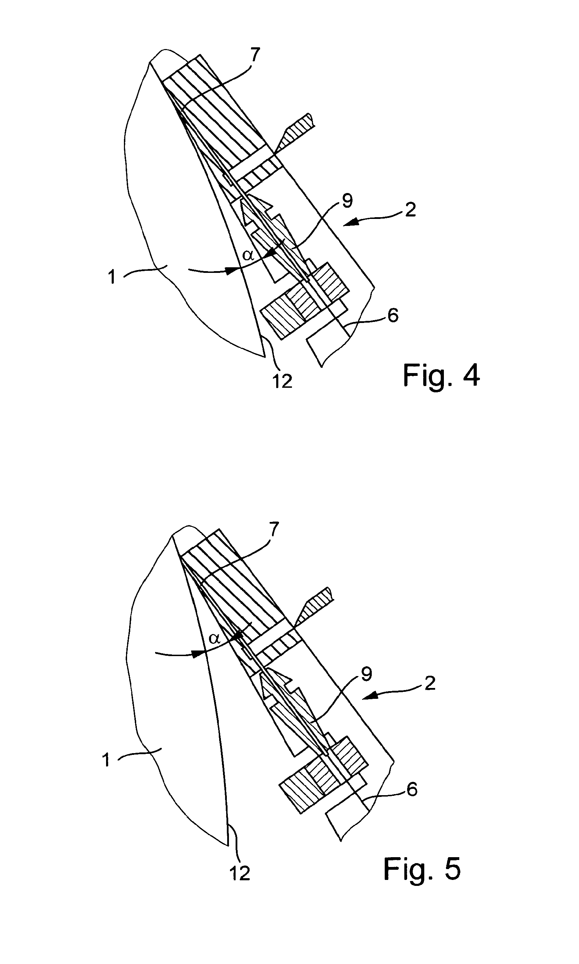

Anhand der Figuren 4, 5 und 6 wird der Herstellungsprozess beschrieben. Figur 4 zeigt die Position der Dreh- und Positioniereinrichtung 2 in Bezug auf die Schleifeinrichtung 1 während des ersten Schleifvorgangs. Der Lanzettendraht 6 ist von der Transporteinrichtung 3 der Dreh- und Positioniereinrichtung 2 zugeführt worden und in dem Drehelement 9 fixiert. Der Neigungswinkel zwischen der Schleiffläche 12 und dem freien Ende 7 des Lanzettendrahtes 6 beträgt hier 7,5°. Die Schleifscheibe 11 schleift das freie Ende 7 des Lanzettendrahtes 6 unter dem Neigungswinkel, so dass eine erste Schlifffläche 31 erzeugt wird, die als Grundschlifffläche bezeichnet wird und bevorzugt in einem Winkel von 7,5° zur Längsachse des Lanzettendrahtes 6 verläuft.

Während des Schleifvorganges wird das freie Ende 7 des Lanzettendrahtes 6 von dem Stützelement 21 gestützt, welches Bestandteil der Dreh- und Positioniereinrichtung 2 ist. Das freie, zu schleifende Ende des Lanzettendrahtes 6 liegt an der Stützfläche 22 an, so dass es nicht von dem Schleifelement 10 verbogen werden kann und glatte, ebene Schliffflächen entstehen.

Bevorzugt hat bei mindestens einem der Schleifvorgänge der Neigungswinkel α zwischen der Schleiffläche 12 und der Achse des Lanzettendrahtes 6 einen Wert zwischen 5° und 10°. Besonders bevorzugt liegt der Neigungswinkel α zwischen 7° und 8°.

Nachdem alle Lanzettendrähte 6 in der Dreh- und Positioniereinrichtung 2 in einem ersten Schleifvorgang geschliffen worden sind, so dass sie die Grundschlifffäche 32 aufweisen, wird die Dreh- und Positioniereinrichtung 2 verschwenkt und der Neigungswinkel α verändert. Bevorzugt liegt der neue Neigungswinkel α zwischen der Schleiffläche 12 und der Achse des Lanzettendrahtes 6 zwischen 15° und 25°, besonders bevorzugt zwischen 17° und 20°. Dieser Neigungswinkel α ist bei mindestens einem der anderen Schleifvorgänge eingerichtet, bevorzugt bleibt er bei dem dritten Schleifvorgang unverändert. In dem in Figur 5 gezeigten Beispiel ist der Neigungswinkel α gleich18°.

Bevor die Schleifeinrichtung 1 mit dem nächsten Schleifvorgang beginnt, um eine weitere Schlifffläche 33 zu erzeugen, wird der Lanzettendraht 6 von dem Drehelement 9 um seine Längsachse gedreht. Bevorzugt ist dabei eine Drehung mit einem Drehwinkel β zwischen ca. 15° und ca. 25°, besonders bevorzugt ist ein Drehwinkel von β = 20°.

Am Ende des zweiten Schleifvorgangs weisen die freien Enden der Lanzettendrähte 6 neben der Grundschlifffläche 32 eine weitere Schlifffläche 33 auf, die als erste Schneidfläche bezeichnet wird.

Im Anschluss an den zweiten Schleifvorgang werden alle Lanzettendrähte 6 der Dreh- und Positioniereinrichtung 2 durch die jeweiligen Drehelemente 9 um ihre eigene Achse gedreht. Diese zweite Drehung erfolgt entgegen der Drehrichtung der ersten Drehung. Der Drehwinkel β' liegt bevorzugt zwischen ca. 30° und ca. 50°, besonders bevorzugt bei 40°. Der Drehwinkel β' entspricht dem Doppelten des Drehwinkels β der ersten Drehung.

Nach der Drehung aller Lanzettendrähte 6 wird die Schleifeinrichtung 1 wieder an der Dreh- und Positioniereinrichtung 2 vorbeigeführt, so dass in einem dritten Schleifvorgang eine weitere Schlifffläche 33 an dem freien Ende 7 der Lanzettendrähte 6 erzeugt wird. Als Ergebnis dieses dritten Schleifvorgangs weist das freie Ende 7 des Lanzettendrahtes 6 neben der Grundschlifffläche 32 und der ersten Schneidfläche 34 eine zweite Schneidfläche 35 auf.

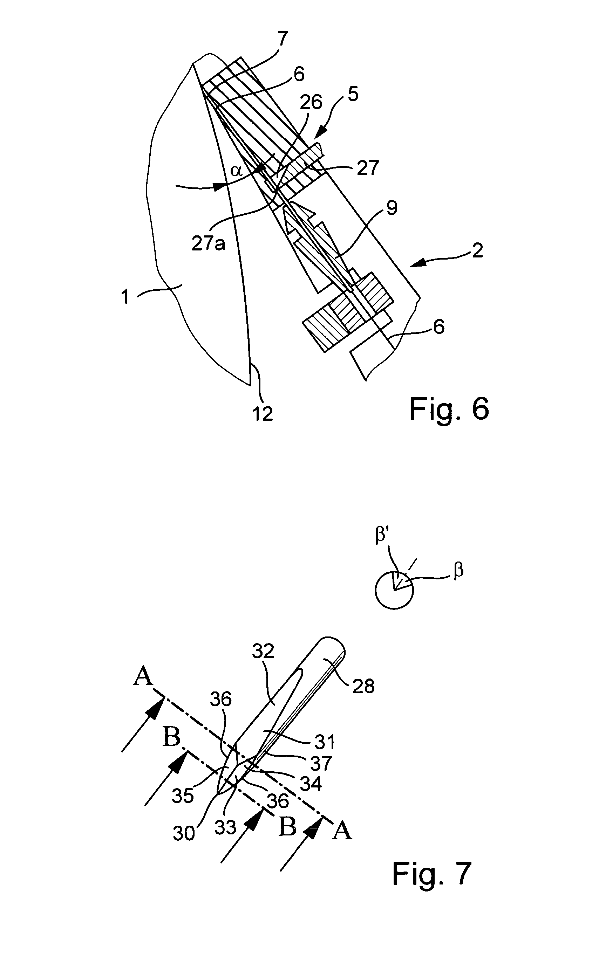

Nachdem alle freien Enden der in der Dreh- und Positioniereinrichtung 2 eingespannten Lanzettendrähte 6 geschliffen worden sind und alle Enden einen Dreifacettenschliff haben, wird das Schneidmesser 27 der Trenneinheit 25 in der Nut 26 auf den Lanzettendraht 6 zu geführt (Fig. 6). Das Schneidmesser 27 befindet sich an der Trennposition 27a und trennt das freie Ende 7 des Lanzettendrahtes 6 an einer Stelle zwischen seiner Spitze 30 und dem Drehelement 9 ab. Dadurch entsteht die in Figur 7 gezeigte Lanzette 28 mit einer Länge von 12 mm und einer geschliffenen scharfen Spitze 30, die einen Einstich in der Haut eines Körperteils auf schmerzarme Weise erzeugt.

Die Lanzette 28 hat den gewünschten Dreifacettenschliff mit einer Grundschlifffläche 32 und zwei Schneidflächen 34, 35. Die beiden Schneidflächen 34, 35 bilden mit der Mantelfläche 37 eine Schneide 36, mit der beim Einstich der Lanzette 28 in die Haut eines Körperteils die Wunde aufgeschnitten wird, so dass nach Herausziehen der Lanzette Blut aus der Wunde austreten kann.

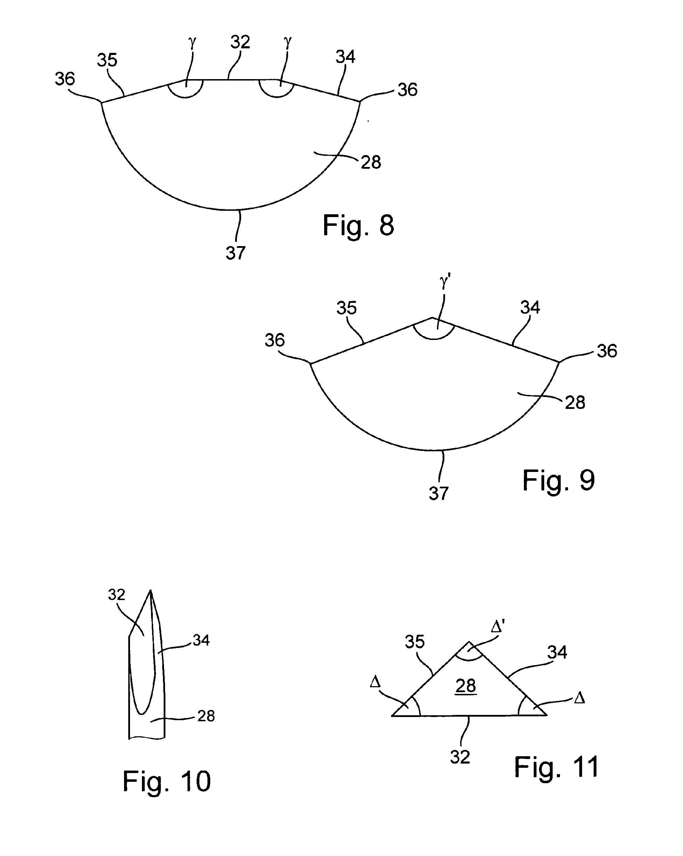

Die in den Figuren 8,9 und 11 gezeigten Schnittwinkel stellen den Winkel zwischen den in einer zur Längsachse der Lanzette 28 senkrechten Schnittebene liegenden Schnittlinien der entsprechenden Flächen dar. Die gezeigten Winkel γ, γ', Δ und Δ' werden also zwischen den sich bei einem Schnitt senkrecht zur Längsachse des Lanzettendrahts ergebenen Schnittlinien der entsprechenden Flächen gebildet.

Figur 8 zeigt ein Querschnitt durch die Lanzette entlang der Linie A-A. Die Lanzette 28 weist je einen Winkel γ zwischen der Grundschlifffläche 32 und den Schneidflächen 34 bzw. 35 auf. Der Winkel γ beträgt 180°-β, wobei β der Drehwinkel ist. In dem gezeigten Beispiel ist der Winkel γ = 160°.

Deutlich zu erkennen sind die beiden Schneiden 36, die jeweils zwischen der Schneidfläche 34 bzw. 35 und der Mantelfläche 37 gebildet werden.

In Figur 9 ist ein weiterer Schnitt durch die Lanzette 28 dargestellt. Dieser Schnitt befindet sich näher bei der Spitze der Lanzette 28. Die beiden Schneidflächen 34 und 35 stoßen hier direkt aneinander und bilden einen Winkel γ'. Der Winkel γ' berechnet sich zu γ' = 180° -2β bzw. γ' = 180° -β', wobei β der Drehwinkel der ersten Drehung und β' der Drehwinkel der zweiten Drehung des Drehelements 9 um die Achse des Lanzettendrahts 6 ist. In dem hier dargestellten Beispiel ist der Winkel γ' = 140°.

Neben den in den Figuren 7 bis 9 beschriebenen Lanzettenform ist es mit dem erfindungsgemäßen Verfahren auch möglich, Lanzetten 28 herzustellen, bei denen die Grundschlifffläche 32 den beiden Schneidflächen 34, 35 gegenüberliegt. Eine derartige Lanzette ist in Figur 10 dargestellt.

Zur Verdeutlichung der Anordnung der Grundschlifffläche 32 und der Schneidflächen 33,34 ist in der Figur 11 ein Schnitt durch die Lanzette 28 gemäß Figur 10 dargestellt. Zwischen der Grundschlifffläche 32 und den Schneidflächen 34,35 wird jeweils ein Winkel Δ gebildet, der in dem gezeigten Beispiel etwa 20° beträgt. Zur Herstellung einer Lanzette der hier gezeigten Form wird nach dem Schleifen der Grundschlifffläche 32 der Lanzettendraht 6 um ca. 160° bzw. 200° gedreht, bevor die Schneidfläche 34 bzw. 35 geschliffen wird. Im Anschluss an das Schleifen der Schneidfläche 34 bzw. 35 wird der Lanzettendraht 28 nochmals um 40° gedreht. Dadurch entsteht ein Winkel Δ' von 140° zwischen den Schneidflächen 34,35. Der Winkel Δ' ergibt sich aus 180° minus dem Drehwinkel, um den der Lanzettendraht zwischen den beiden Schleifvorgängen gedreht wird. Selbstverständlich sind auch andere Δ möglich, die bevorzugt im Bereich von 10° bis 30°, besonders bevorzugt im Bereich von 15° bis 25° liegen.

Diese als "Hinterschliff-Lanzette" bezeichnete Schliffform ist deshalb möglich, weil sich der Lanzettendraht um ca. 270° um seine eigene Achse drehen lässt, ohne dass ein Durchrutschen des Lanzettendrahtes in den Drehelementen 9 zu befürchten ist.

Zur Herstellung der Lanzetten wird Lanzettendraht verwendet, der aus Metall, vorzugsweise Stahl besteht. Auch Legierungen von Metallen sind geeignet. Der Durchmesser des Lanzettendrahtes beträgt ca. 0,2 bis 0,8 mm, bevorzugt 0,3 mm.

## Patentansprüche

1. Verfahren zum Herstellen von Lanzetten zur Entnahme von Blut für medizinisch-analytische Zwecke, die eine Schlifffläche (31) aufweist, welche in wenigstens einem Schleifvorgang erzeugt wird, wobei
ein Lanzettendraht (6) mittels einer Positioniereinrichtung (2a) in einer definierten Schleifposition relativ zu einem Schleifelement (10) einer Schleifeinrichtung (1) positioniert wird,
**dadurch gekennzeichnet, dass** die folgenden Verfahrensschritte in dieser Reihenfolge durchgeführt werden:
- Abziehen des Lanzettendrahtes (6) von einer Rolle (5) und transportieren in die Positioniereinrichtung (2a),
- Fixieren des Lanzettendrahtes (6) in der Positioniereinrichtung (2a),
- Schleifen des freien Endes (7) des Lanzettendrahtes (6) mittels der Schleifeinrichtung (1) zum Erzeugen der Schlifffläche (31), und
- Abtrennen des freien Endes (7) des Lanzettendrahtes (6) in einer Trennposition zu einer Lanzette (28) mit definierter Länge.

2. Verfahren nach Anspruch 1, **dadurch gekennzeichnet, dass** zum Herstellen von Lanzetten mit einer geschliffenen Spitze, die die Schlifffläche (31) und mindestens eine weitere Schlifffläche (33), die in einem definierten Winkel zueinander orientiert sind, aufweisen, das freie Ende (7) des Lanzettendrahtes (6) mittels der Schleifeinrichtung (1) zum Erzeugen der Mehrzahl von Schliffflächen (31,33) geschliffen wird, wobei
- in einer Mehrzahl von Schleifvorgängen jeweils eine der Schliffflächen (31,33) erzeugt wird,
- der Lanzettendraht (6) zwischen den Schleifvorgängen in eine der nächsten zu erzeugenden Schlifffläche (33) entsprechende Schleifposition gedreht wird, und
- die Drehbewegung eine axiale Drehbewegungskomponente hat.

3. Verfahren nach Anspruch 2, **dadurch gekennzeichnet, dass** der Neigungswinkel α des freien Endes (7) des Lanzettendrahtes (6) relativ zu der Schleiffläche (12) des Schleifelementes (10) zwischen mindestens zwei der Schleifvorgänge verändert wird.

4. Verfahren nach einem der Ansprüche 1 bis 3, **dadurch gekennzeichnet, dass** die Positioniereinrichtung (2a) ein Drehelement (9) aufweist, in dem der Lanzettendraht (6) fixiert und mittels dessen er um seine Längsachse gedreht wird.

5. Verfahren nach Anspruch 4, **dadurch gekennzeichnet, dass** mindestens ein Teil des Drehelementes (9) in Längsrichtung des Lanzettendrahtes (6) in mindestens drei Segmente (18) segmentiert ist und der Lanzettendraht (6) durch Zusammendrücken der Segmente (18) in Richtung auf die Längsachse des Lanzettendrahtes (6) fixiert wird.

6. Verfahren nach einem der Ansprüche 4 oder 5, **dadurch gekennzeichnet, dass** der Lanzettendraht (6) an einer Stelle abgetrennt wird, die zwischen dem Drehelement (9) und der geschliffenen Spitze (30) des Drahtes liegt.

7. Verfahren nach einem der Ansprüche 4 bis 6, **dadurch gekennzeichnet, dass** das Abtrennen des freien Endes (7) des Lanzettendrahtes (6) erfolgt, während der Lanzettendraht (6) in dem Drehelement (9) fixiert ist.

8. Verfahren nach einem der vorhergehenden Ansprüche, **dadurch gekennzeichnet, dass** bei mindestens einem der Schleifvorgänge der Neigungswinkel α zwischen der Schleiffläche (12) und der Längsachse des Lanzettendrahtes (6) zwischen 5° und 10°, vorzugsweise zwischen 7° und 8° beträgt.

9. Verfahren nach Anspruch 8, **dadurch gekennzeichnet, dass** der Neigungswinkel α zwischen der Schleiffläche (12) und der Längsachse des Lanzettendrahtes (6) bei mindestens einem der anderen Schleifvorgänge zwischen 15° und 25°, vorzugsweise zwischen 17° und 20° beträgt.

10. Verfahren nach einem der vorhergehenden Ansprüche, **dadurch gekennzeichnet, dass** das freie Ende (7) des Lanzettendrahtes (6) während der Schleifvorgänge von einem Stützelement (21) gestützt wird, welches Bestandteil der Positioniereinrichtung (2a) ist.

11. Verfahren nach einem der vorhergehenden Ansprüche, **dadurch gekennzeichnet, dass** die Positioniereinrichtung (2a) zur Aufnahme einer Mehrzahl von Lanzettendrähten (6) ausgebildet ist und die Lanzettendrähte (6) nebeneinander in einer quer zur Längsrichtung ihrer freien Enden (7) verlaufenden Ebene fixiert sind.

12. Verfahren nach einem der vorhergehenden Ansprüche, **dadurch gekennzeichnet, dass** das Schleifelement (10) während eines Schleifvorganges derartig bewegt wird, dass die freien Enden (7) von mehreren in der Positioniereinrichtung (2a) fixierten Lanzettendrähten (6) während der Bewegung nacheinander geschliffen werden.

13. Verfahren nach einem der vorhergehenden Ansprüche, **dadurch gekennzeichnet, dass** die freien Enden (7) von mehreren der Positioniereinrichtung (2a) fixierten Lanzettendrähten (6) gleichzeitig von dem Schleifelement (10) geschliffen werden.

14. Verfahren nach einem der vorhergehenden Ansprüche, **dadurch gekennzeichnet, dass** das freie Ende (7) des Lanzettendrahtes (6) derartig abgetrennt wird, dass die Länge der resultierenden Lanzette (28) von deren Spitze (30) bis zu der Trennstelle höchstens 20 mm, vorzugsweise höchstens 16 mm, besonders bevorzugt höchstens 10 mm beträgt.

15. Verfahren nach einem der vorhergehenden Ansprüche, **dadurch gekennzeichnet, dass** das freie Ende (7) des Lanzettendrahtes (6) mittels eines Hackmessers, eines Schneidmessers (27), einer Trennscheibe oder eines Lasers abgetrennt wird.

16. Verfahren nach einem der Ansprüche 1 bis 15, **dadurch gekennzeichnet, dass** zwischen dem Schleifen einer als Grundschlifffläche (32) ausgebildeten Schlifffläche (31) und einer weiteren Schlifffläche (33) das freie Ende (7) des Lanzettendrahtes (8) um einen definierten Drehwinkel gedreht wird, der im Bereich von 90° bis 270°, bevorzugt in einem Bereich von 150° bis 210° und besonders bevorzugt im Bereich von 160° bis 170° und von 190° bis 200° liegt..

17. Lanzette aus Lanzettendraht (6), hergestellt nach einem der Ansprüche 1 bis 16.

18. Lanzette nach Anspruch 17, **dadurch gekennzeichnet, dass** sie eine geschliffene Spitze (30) aufweist und ihre Länge höchstens 16 mm, vorzugsweise höchstens 12 mm, besonders bevorzugt höchstens 5 mm beträgt.

19. Lanzette mit einer geschliffenen Spitze (30) aus Lanzettendraht (6), **gekennzeichnet durch** eine Grundschlifffläche (32) und zwei Schneidflächen (34,35), wobei die Grundschlifffläche (32) mit jeder Schneidfläche (34,35) je einen Winkel Δ bildet, der kleiner als 90°, bevorzugt kleiner als 30°, besonders bevorzugt kleiner als ca. 20° ist.
